# EUROPÄISCHE PATENTSCHRIFT

(11) **EP 0 936 903 B1**
(45) Veröffentlichungstag und Bekanntmachung des Hinweises auf die Patenterteilung: **08.10.2003**
(21) Anmeldenummer: 96931056.4
(22) Anmeldetag: 07.09.1996
(51) Int. Cl.: A61K 9/22

(54) **MITTEL MIT RETARDWIRKUNG**
PRODUCT WITH EXTENDER EFFECT
PRODUIT A EFFET RETARD

(43) Veröffentlichungstag der Anmeldung: 25.08.1999
(73) Patentinhaber: Beisel, Günther, 40789 Monheim (DE)
(72) Erfinder: Beisel, Günther, 40789 Monheim (DE)
(74) Vertreter: Fitzner, Uwe, Dr.
(86) Internationale Anmeldenummer: EP9603950
(87) Internationale Veröffentlichungsnummer: WO98009617

(56) Entgegenhaltungen:
- EP-A- 0 170 979
- EP-A- 0 471 217
- EP-A- 0 669 129
- WO-A-91/17745
- DE-A- 4 419 818
- US-A- 3 435 110

## Beschreibung

Die vorliegende Erfindung betrifft eine Weiterentwicklung auf dem Gebiet der Wirkstoffsysteme mit zeitverzögerter und schonender Freigabe von Wirkstoffen aus einem Trägermaterial, insbesondere aus dem Bereich medizinischer und/oder biologischer Wertstoffe. Kombinationsmaterialien dieser Art - und zwar sowohl aus dem Bereich pharmazeutischer Hilfsstoffe als auch biologischer Präparate, beispielsweise Pflanzenschutzmittel - sind Gegenstand zahlreicher Untersuchungen und druckschriftlicher Veröffentlichungen. Sie werden beispielsweise unter den Begriffen Retard-Systeme, Depot- bzw. Slow-Release-Materialien oder ganz allgemein als Mittel mit verzögerter Wertstofffreigabe bezeichnet.

Die Retardwirkung kann beispielsweise durch ein Umhüllungsverfahren bewirkt werden, bei welchem ein Granulat oder eine bereits geformte Tablette mit einer Hüllschicht überzogen wird, durch die der Wirkstoff über einen längeren Zeitraum hindurch defundiert (vgl. z.B. DE-OS 4239244). Um eine möglichst kontrollierte Wirkstofffreigabe zu erreichen, beschreibt die US-PS 3916899 beispielsweise eine Retardierungsform für Wirkstoffe, bei welcher das Wirkstoffreservoir von einer semipermeablen Lackschicht umgeben wird, welche für den Wirkstoff undurchlässig, für die jeweilige Flüssigkeit des Umgebungsmediums jedoch permeabel. Die umgebende Lackschicht enthält zusätzlich eine geometrisch wohl definierte Öffnung, durch welche der in der Umgebungsflüssigkeit gelöste Wirkstoff nach außen gelangen kann. Nach Einnahme des Mittels defundieren die Verdauungskräfte durch die semipermeable Lackschicht hindurch in das Innere der Kapsel. Der Wirkstoff im Inneren der Kapsel wird in der einströmenden Flüssigkeit kontinuierlich gelöst und gelangt über die geometrische Öffnung in der Lackschicht mit definierter Rate nach außen.

Bei diesem System ist es doch immer wieder im Magen- oder Darmtrakt, je nach eingesetzter Kapselfüllung zu lokalen Irritationen des Gewebes durch erhöhte Konzentrationen gekommen. Ein Teil des Wirkstoffes verbleibt außerdem in der Regel in der Arzneiform und steht somit nicht für die gewünschte Resorption zur Verfügung. Außerdem ist die Herstellung der Retadierungsform sehr aufwendig, da die Lackschicht mit den definierten Öffnungen versehen werden muß.

Nach anderen Verfahren bestehen die Depotmaterialien aus einem Träger mit oder ohne Eigenwirkung, in den der zeitverzögert freizusetzende Wertstoff eingearbeitet ist. Besondere Beachtung finden in der Literatur der letzten Jahre als Trägermaterial Polymerverbindungen auf der Basis von Polyestem aus niederen Hyroxycarbonsäuren mit insbesondere 2-6 C-Atomen im Hydroxycarbonsäuremoiekül. Trägermaterialien dieser Art sind ihrerseits Hydrolyse-labil und unterliegen biologischen Mechanismen.

Aus der jüngeren einschlägigen Literatur sei beispielsweise verwiesen auf die US-PS 4011312, die feste Zubereitungsformen von Co-Polyestem aus Glycolsäure und Milchsäure mit Molekulargewichten von unter 2.000 als Trägermaterial in Abmischung mit antibiotischen Wirkstoffen wie Tetracyclin, Neomyzin und anderen Antibiotika offenbart. Zahlreiche Untersuchungen beschäftigen sich mit der Verwendung von resorbierbaren Potyestern auf Basis Glycolsäure/Milchsäure als Trägermaterial mit verzögerter Wirkstofffreisetzung (vgl. z.B. D.L. Wiese et al. in: Drugs Carriers in Medicine Academic Press London 1979, Seiten 237-270).

Eine spezielle Art der Retardierung mittels trägergebundener Wirkstoffe offenbart die DE OS 4413350. Dort wird eine Retardform beschrieben, bei der der Wirkstoff in eine Mischung eines wasserunlöslichen polymeren, eines Lipids sowie eines kolloidal hochviskos-wasserlöslichen, gelbildenden oder zumindest wasserquellbaren Polymeren eingebettet ist. Das Grundprinzip der dort beschriebenen Polymermatrix ist eine durch geeignete lipophile Substanzen klassifizierte Matrix aus einem in Wasser und gastrointestinalen Flüssigkeiten unlöslichen Polymeren. In diese Matrix aus unlöslichem Polymer und lipophiler Komponente ist zusätzlich ein Gelbildnder, also ein in Wasser hochviskoslösliches oder zumindest quellbares Polymer eingebaut. Dieser Gelbildner bewirkt ein Aufbrechen der Retardmatrix durch die Quellung bei Berührung mit der Magenflüssigkeit, so daß der Wirkstoff freigesetzt werden kann.

Nachteilig bei diesem Aufbau einer Retardform ist die sehr komplizierte Herstellungsweise, insbesondere die Abstimmung der verschiedenen für die Retardwirkung verantwortlichen Komponenten. Hinzu kommt, daß alle bisher beschriebenen Retardformen in Form kleinvolumiger Gebilde in den Magen gelangen und sich dort deren Volumen in der Regel auch nicht wesentlich vergrößert. Die Arzneimittel können sich daher in Falten des Darms und des Magens ablagern. Die Folge können Irritationen oder sogar Perforationen der Magen- und Darmwände sein. Außerdem kommt es bei den bisherigen kleinvolumigen Medikamenten nicht zu der erforderlichen gleichförmigen Verteilung der zeitverzögert freigesetzten Wirkstoffe im Magen- und Darmtrakt.

Aufgabe der vorliegenden Erfindung ist es demgemäß, ein Mittel mit zeitverzögerter und schonender Freisetzung der Wirkstoffe zur Verfügung zu stellen, enthaltend ein in Wasser und gastrointestinalen Flüssigkeiten unlösliches oder zeitlich verzögert lösliches, quellendes Trägermaterial, Wirkstoffe sowie ggf. übliche Hilfsstoffe und ggf. eine in Wasser und gastrointestinalen Flüssigkeiten lösliche Umhüllung, das die geschilderten Nachteile der bisherigen Retardformen nicht aufweist. Insbesondere soll das Mittel durch seine großvolumige Form verhindem, daß es durch eine Ablagerung an den Magen-Darmwänden zu lokalen Irritationen kommt. Darüber hinaus soll eine möglichst großflächige Verteilung der freigesetzten Wirkstoffe im Magen-Darmtrakt erreicht werden.

Diese Aufgabe wird durch ein Mittel gelöst, das erhältlich ist, indem ein großvolumiges, schwammförmiges Gebilde eines mit Wirkstoff beaufschlagten Trägermaterials zum Teil in verschiedene Formen komprimiert und ggf. mit der Umhüllung versehen wird.

Unter schwammartigen Gebilden sind erfindungsgemäß Schäume zu verstehen, die aus gasgefüllten, kugel- polyätherförmigen Zellen bestehen, welche durch hochviskose oder feste Zellstege begrenzt sind. Einsetzbar sind erfindungsgemäß sowohl natürlich vorkommende Schwämme als auch synthetisch hergestellte schwammartige Gebilde.

Die Herstellung der schwammartigen bzw.-förmigen Gebilde erfolgt mit an sich bekannten Methoden nach dem Stand der Technik. In Abhängigkeit von dem eingesetzten Ausgangsmaterial kann im einfachsten Falle ein Schaum durch Einblasen,durch Schlagen, Schütteln, Verspritzen oder Rühren in der betreffenden Gasatmosphäre erhalten werden. Bei den Polymeren entsteht die Schaumstruktur aufgrund chemischer Reaktionen. So werden bei den Polyurethanen durch Zugabe von Blähmitteln, die sich bei bestimmter Temperatur während der Verarbeitung unter Gasbildung zersetzen, oder durch Zusatz von flüssigen Lösemitteln während der Polymerisation geschäumt. Die Verschäumung erfolgt entweder beim Verlassen des Extrusionswerkzeuges, d.h. im Anschluß an das Extrudieren oder Spritzgießen oder in offenen Formen. Die Härtung erfolgt unter den für die jeweilige chemische Verbindung des Trägermaterials charakteristischen Bedingungen.

Unabdingbare Voraussetzung für die Einsetzbarkeit des erfindungsgemäßen Trägermaterials und der Schwammstruktur ist, daß das Material komprimierbar ist, ohne daß die Zellstege brechen. Um das erfindungsgemäße Trägermaterial nämlich für ein Mittel einsetzen zu können, muß das schaumförmige bzw.-artige Trägermaterial auf eine für die orale oder enterale Verabreichung geeignete Größe komprimiert werden. Üblicherweise wird das Trägermaterial auf ein Volumenvon höchstens 2 cm³ komprimiert. Für die üblichen Darreichungsformen sollte das Volumen unter 3 cm³ liegen.

Die Komprimierung besteht darin, das Trägermaterial zu pressen oder in ähnlicher Weise zu komprimieren, wodurch dieses auf einen Bruchteil seines freien Volumens reduziert wird. Das Trägermaterial wird hierzu vorher zweckmäßigerweise in seine Endgröße geschnitten. Sofern das erfindungsgemäße Mittel in Form von Kapseln verabreicht werden soll, kann der so hergestellte Press-Block in einer Kapsel verschlossen werden.

Ebenso kann der Press-Block mit einer Lackschicht oder anderen Schutzschichten, die sich erst unter Einfluß der Margen- und Darmflüssigkeit auflöst, überzogen werden.

Für die Auswahl des Trägermaterials und die Art der Schaumbildung ist schließlich auch wesentlich, daß das Material quellfähig bleibt, ohne daß die Zellstege zerstört werden. Unter physiologischen Bedingungen soll das komprimierte Trägermaterial sich vorzugsweise auf das Zwei-bis Zehnfache, besonders bevorzugt auf das Vier-bis Achtfache seines Volumens ausdehnen können.

Die Wirkstofffreisetzungsflächen des unter physiologischen Bedingungen vergrößerten Trägermaterials betragen erfingungsgemäß 15 bis 25 cm². Im Vergleich dazu liegen die Werte der Freisetzungsflächen nach dem Stand der Technik bei 0,5 bis 1,5 cm².

Als Trägermaterial können erfindungsgemäß natürliche, halbsynthetische oder synthetische Polymere zum Einsatz kommen. Beispiele geeigneter synthetischer Polymere sind Polyurethane, Polyacrylate, Poly(met)acrylsäureester, Homo- und Copolymere des Vinylacetats. Zu den natürlichen und halbsynthetischen Polymeren zählen u.a. Cellulose, Ether, Diethylcellulose oder Celluloseester, wie Cellulosediacetat, Cellulosetriacetat, Celluloseacetat-Propionat und Celluloseacetat, Butyrat. Erfingungsgemäß geeignet sind z.B. Cellulosederivate, insbesondere entsprechende Ether, z.B. Methylcellulose, Hydroxypropylcellulose, Hydroxypropyl-methylcellulose, oder Natriumcarboxymethylcellulose (vorzugsweise solche Verbindungen mit höherer Viskosität); gewisse Polymere, wie Polyacrylsäure und Salze davon; natürliche (anionische) Schleimstoffe, z.B. Xanthan Gummi, Guar Gummi, Traganth oder Alginsäure und Salze davon, und dergleichen Darüber hinaus ist auch der Einsatz unlöslicher Polysacharide, wie Chitin bzw. Chitinderivate oder mikrokristalliner Cellulose denkbar. Erfindungsgemäß besonders bevorzugt werden linerare hochmolekulare Polymere. Vor allem sind erfindungsgemäß solche Polymere einsetzbar, die Faserstruktur besitzen. Beispiele für solche Stoffe sind die Skleroproteine, wie Keratine, Conchagene, Fibroin, Elastine, Chitin und Collagen. Letzteres wird erfindungsgemäß besonders bevorzugt.

Vor dem Komprimieren erfolgt zweckmäßigerweise die Beaufschlagung mit Wirkstoffen. Dies kann vor, während oder nach der Herstellung des schwammartigen Gebildes erfolgen. Für die Beaufschlagung mit Wirkstoff kommen alle üblichen Methoden Betracht. Im einfachsten Falle kann dies während der Herstellungsphase des Schwammaterials durch Mischen von Trägermaterial und Wirkstoff erfolgen. Anschließend wird getrocknet und die beschriebene Komprimierung durchgeführt.

Die Wirkstoffmenge pro Dosis-Einheit und die Konzentration können je nach Wirksamkeit und gewünschter Freisetzungsgeschwindigkeit in weiten Grenzen variieren. Die einzige Bedingung ist, daß sie zur Erzielung der gewünschte Wirkung und Zielsetzung ausreichen. So kann die Wirkstoffkonzentration im Bereich von 0,1 bis 87, vorzugsweise 1 bis 80, insbesondere 1-75 Gew.-% liegen.

Unter Wirkstoffen im Sinne der Erfindung sind alle Stoffe mit einer pharmazeutischen oder biologischen Wirkung. Beispiele sind Betamethason, Thioetsäure, Sotalol, Salbutamol, Norfenefrin, Solymarin, Dihydroergotamin, Buflumedil, Etofibrat, Indometacin, Oxazepam, beta-Acetyldigoxim, Piroxicam, Haloperidol, ISMN, Amitirptylin, Diclofenac, Nifedipin, Verapamil, Pyritinol, Nitrendipin, Doxycyclin, Bromhexin, Methylprednisolon, Clonidin, Fenofibrat, Allopurinol, Pirenzepin, Levothyroxin, Tamoxifen, Metildigoxin, o-(beta-Hydroxyethyl)rutosid, Propicillin, Aciclovir-mononitrat, Paracetamol, Naftidrofuryl, Pentoxyfyllin, Propafenon, Acebutolol, L-Thyroxin, Tramadol. Bromocriptin, Loperamid, Ketotifen, Fenoterol, Ca-Dobelisat, Propranolol, Minocyclin, Nicergolin, Ambroxol, Metoprolol, beta-Sitosterin, Enalaprilhydrogenmaleat, Benzafibrat, ISDN, Gallopamil, Xantinolnicotinat, Digitoxin, Flunitrazepan, Bencyclan, Dexapanthenol, Pindolol, Lorazepam, Diltiazem, Piracetam, Phenoxymethylpenicillin, Furosemid, Bromazepam, Flunarizin, Erythromycin, Metoclopramid, Acemetacin, Ranitidin, Biperiden, Metamizol, Doxepin, Dikalium-Chlorazepat, Tetrazepam, Estramustinphosphat, Terbutalin, Captopril, Maprotilin, Prazosin, Atenolol, Glibendamid, Cefaclor, Etilefrin, Cimetidin, Theophyllin, Hydromirphon, Ibuprofen, Primidon, Clobazam, Oxaceprol, Medroxyprogresteron, Flecainid, Mg-Pridoxal-5-phosphatglutaminat. Hymechromon, Etofyllindofibrat, Vincamin, Cinarizin, Diazepam, Ketoprofen, Flupentixol, Molsidomin, Glibomuid, Dimetinden, Melperon, Soquinolol, Dibydrocodein, Clomethiazol, Clemastin, Glisoxepid, Kallidinogenase, Oxyfedrin, Baclofen, Carboxymethylcystein, Thiorodacin, Betathistin, L-Tryptophan, Myrtol, Bromalaine, Prenylamin, Salazosulfapyridin, Astemizol, Sulpirid, Benzerazid, Dibenzepin, Acetylsalicylsäure, Miconazol, Nystatin, Ketonconazol, Na-Picosulfat, Colestyramin, Gemifibrocil, Rifampicin, Fluorocortolon, Mexiletin, Amoxicillin, Terfenadrin, Mucopolysaccharidpolyschwefelsäureester, Triazolam, Mianserin, Tiaprofensäure, Ameziniummetilsulfat, Mefloquin, Probucol, Chinidin, Carbamepin, Mg-L-aspartat, Penbutolol, Piretanid, Amitriptylin, Cyproteron, Na-Valpropinat, Mebeverin, Bisacodyl, 5-Amino-Salicylsäure, Dihydralazin, Magaldrat, Phenprocoumon, Amantadin, Naproxen, Cartelol, Famotidin, Methyldopa, Auranofin, Estriol, Nadolol, Levomepromazin, Doxorubicin, Medofenoxat, Azathioprin, Flutamid, Norfloxacin, Fendilin, Prajmaliumbitartrat, Aescin.

Weitere Beispiele sind folgende Wirkstoffe: Acetaminophen (= Paracetamol), Acetohexamid, Acetyldigoxim, Acetylsalicylsäure, Acromycin, Anipamil, Benzocain, beta-Carotin, Choramphenicol, Chlordiazepoxid, Chlormadinoacetat, Chlorthiazid, Cinnarizin, Clonazepam, Codein, Decamethason, Diazepam, Dicumarol, Digitoxin, Digoxin, Dihydroergotamin, Drotaverin, Flunitrazepam, Furosemid, Gramiddin, Griseofluvin, Hexobarbital, Hydrochlorothiazid, Hydrocortison, Hydroflumethazig, Indimethazin, Ketoprofen, Lonetil,, Medazepam, Mefrusid, Methandrostenolon, Methylprednisolon, Methylsulfadiazin (= Sulfaperin), Nalidixinsäure; Nifedipin, Nitrazepam, Nitrofurantoin, Nystatin, Ostradiol, Papaverin, Phenacetin, Phenobarbital, Phenylbutazon, Phenytoin, Prednison, Reserpin, Spironolacton, Streptomycin, Sulfadimidin (= Sulfamethazin), Sulfamethizol, Sulfamethoxazol (= Sulfameter), Sulfaperin, Sulfathiazol, Sulfisoxazol, Testosteron, Tolazamid, Tolbutamid, Trimethoprim, Tyrothricin, Vitamine, Mineralien.

Neben den genannten Wirkstoffen können dem Trägermaterial auch weitere Hilfsstoffe beigefügt werden. Unter anderem können noch zusätzlich retardierende Stoffe in Frage kommen.

Als retardierende Hilfsstoffe können im wesentlichen wasserunlösliche Hilfsstoffe oder Gemische davon, wie Lipide, u.a. Fettalkohole, z.B. Cetylalkohol, Stearylalkohol und Cetostearylalkohol; Glyceride, z.B. Glycerinmonostearat oder Gemische von Mono-, Di- und Triglyceriden pflanzlicher Öle; hydrierte Öle, wie hydriertes Rizinusöl oder hydriertes Baumwollsamenöl; Wachse, z.B. Bienenwachs oder Carnaubawachs; feste Kohlenwasserstoffe, Z.B. Paraffin oder Erdwachs; Fettsäuren, z.B. Stearinsäure; gewisse Cellulosederivate, z.B. Ethylcellulose oder Acetylcellulose; Polymere oder Copolymere, wie Polyalkylene, z.B. Polyäthylen, Polyvinylverbindungen, z.B. Polyvinylchlorid oder Polyvinylacetat, sowie Vinylchlorid-Vinylacetat-Copolymere und Copolymere mit Crotonsäure, oder Polymere und Copolymere von Acrylaten und Methacrylaten, z.B. Copolymerisate von Acrylsäureester und Methacrylsäuremethylester, verwendet werden..

Die Abgabe von Wirkstoffen, die im neutralen Darmmilieu nicht besonders gut, im saueren Magenbereich jedoch besser löslich sind, kann auch zusätzlich mittels Stoffen retardiert werden, die funktionelle Carboxylgruppen aufweisen und sich im neutralen Bereich lösen, z.B. Schellack, Celluloseester, z.B. Celluloseacetat-phthalat oder Hydroxypropylmethylcellulosephthalat, oder Halbester von Maleinsäureanhydrid-Copolymeren.

Außer den genannten Hilfsstoffen können die Mittel gemäß der vorliegenden Erfindung zusätzlich Füll- Spreng-, Binde- und Gleitmittel sowie Trägerstoffe enthalten, die auf die Wirkstoffabgabe keinen entscheidenden Einfluß haben. Beispiele sind u.a. Bentonit (Aluminiumoxid-Siliciumoxid-hydrat), Kieselsäure, Cellulose (üblicherweise mikrokristalline Cellulose) oder Cellulosederivate, z.B. Methylcellulose, Natriumcarboxymethylcellulose, Zucker, wie Lactose, Stärken, z.B. Maisstärke oder Derivate davon, z.B. Natriumcarboxymethylstärke, Stärkeleister, Phosphorsäuresalze, z.B. Di- oder Tricalcioumphosphat, Gelatine, Stearinsäure oder geeignete Salze davon, z.B. Magnesiumstearat oder Calciumstearat, Talk, kollodiales Siliciumoxid und ähnliche Hilfsstoffe.

Das mit den Wirkstoffen und Hilfsstoffen beaufschlagte und komprimierte Trägermaterial wird zweckmäßigerweise mit einem Überzug versehen. Im einfachsten Falle handelt es sich um Kapseln, in die das den Wirkstoff enthaltende Trägermaterial eingebracht ist.

Ebenso kann nach üblichen Methoden die Überziehung mit einem Lack erfolgen. Die Lackschicht oder Schutzschicht kann z.B. in einem rotierenden Kessel oder einem Wirbelschichtreaktor aufgesprüht werden. Nach erfolgter Trocknung sind die erfindungsgemäßen Mittel verpackungs- und versandfähig.

Die erfindungsgemäßen Mittel sind in erster Linie für die Zufuhr in Kapsel-, Tabletten-, Dragee-, Zäpfchen- oder anderen herkömmlichen festen Formen in den Körper konzipiert. Die spezielle Art des eingesetzten Trägerstoffes bewirkt, daß dieser sich bei Berührung mit der Magen- bzw. Darmflüssigkeit oder anderen Körperflüssigkeiten ausbreitet und hierbei die in dem aufquellenden schaumförmigen Gebilde vorhandenen Wirkstoffe zeitverzögert und infolge des großen Volumens vorhandenen Flächen entsprechend schonend freigegeben werden. Dadurch, daß das Trägermaterial großvolumig aufquillt, ist gewährleistet, daß sich das erfindungsgemäße Mittel nicht in Falten der Magenoder Darmwand ablagert. Zu den örtlichen Irritationen infolge von stellenweise auftretenden Überkonzentrationen des Wirkstoffes kann es somit nicht mehr kommen. Die entstehende Größe des Trägermaterials gewährleistet vielmehr eine großflächige und somit schonende Verteilung des Wirkstoffs in dem Magen- und Darmtrakt oder anderen Körperhohlräumen.

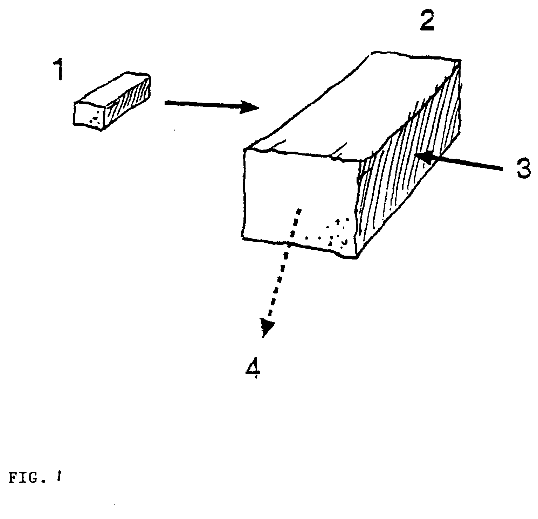

Im folgenden wird die Erfindung unter Bezugnahme auf die Figur näher erläutert:

Zunächst wird der Press-Block 1 hergestellt, der verschiedene Formen haben kann. Dies geschieht erfingungsgemäß vorzugsweise durch Pressen des Trägermaterials. Anschließend wird das gepreßte Trägermaterial applikationsgerecht geschnitten. In dem erfingungsgemäßen Beispiel entsteht so das abgebildete quaderförmige Element 1. Selbstverständlich sind im Rahmen der Erfindung jedoch auch andere Formen eingeschlossen.

Unter Einfluß der Magen - bzw. Darmflüssigkeit entsteht das großvolumige Element 2; Es werden die Wirkstofffreisetzungsflächen 3 gebildet, die sehr viel größer als die aus dem Stand der Technik bekannten Flächen sind. Die Wirkstofffreisetzungsflächen 3 ermöglichen eine gegenüber herkömmlichen Formen erheblich bessere und damit magen - und darmwandschonendere Freisetzung der Wirkstoffe.

Die Wirkstofffreisetzungsflächen 3 haben erfindungsgemäß vorzugsweise eine Größe von 15 bis 25 cm². Nach dem Stand der Technik liegen dagegen die bisherigen Größen der Wirkstofffreisetzungsflächen bei 0,5 bis 1,5 cm². Demgemäß wird durch die erfingungsgemäßen größeren Flächen 3 eine wesentlich bessere und schonendere Wirkstofffreisetzung erreicht. Der in dem Schaum-Trägermaterial eingebettete Wirkstoff 4 wird vor allem kontinuierlich und über Stunden verzögert freigesetzt. Mit den wesentlich kleineren Wirkstofffreisetzungsflächen nach dem Stand der Technik kann nicht annähernd dieselbe Wirkung erzielt werden.

## Patentansprüche

1. Mittel für die zeitverzögerte und schonende Freisetzung von Wirkstoffen im Körper, enthaltend ein in Wasser und gastrointestinalen Flüssigkeiten unlösliches oder schwer lösliches Trägermaterial, Wirkstoffe (4) sowie ggf. weitere Hilfsstoffe und ggf. eine in Wasser und gastrointestinalen Flüssigkeiten/Körperflüssigkeiten lösliche Umhüllung,
**dadurch gekennzeichnet, dass**
die Schaumstruktur des Trägermaterials derart ausgestaltet ist, dass das Material
1. komprimierbar ist, ohne dass die Zellstege brechen und
2. quellbar bleibt, ohne dass die Zellstege zerstört werden.

2. Mittel nach Anspruch 1,
**dadurch gekennzeichnet, dass** vor, während oder nach der Herstellung des schwammartigen Gebildes (1, 2) der Wirkstoff (4) in das Trägermaterial gegeben wird.

3. Mittel nach einem der Ansprüche 1 oder 2,
**dadurch gekennzeichnet, dass** als Trägermaterial Viskoseschwämme eingesetzt werden, deren komprimierte Form (1) in Wasser oder gastrointestinalen Flüssigkeiten/Körperflüssigkeiten auf ein Mehrfaches (2) ihres Volumens (1) quellbar ist.

4. Mittel nach einem der Ansprüche 1 bis 3,
**dadurch gekennzeichnet, dass** das Volumen des komprimierten Trägermaterials (1) höchstens 2 cm³ beträgt.

5. Mittel nach einem der Ansprüche 1 bis 4,
**dadurch gekennzeichnet, dass** als Trägermaterial Viskoseschwämme eingesetzt werden, die in Wasser und gastrointestinalen Flüssigkeiten auf das Zwei-bis Zehnfache, vorzugsweise Vier- bis Achtfache (2) ihres Volumens im gepreßten Zustand (1) quellen.

6. Mittel nach einem der Ansprüche 1 bis 5,
**dadurch gekennzeichnet, dass** das Trägermaterial aus natürlichen, halbsynthetischen oder synthetischen Polymeren besteht

7. Mittel nach einem der Ansprüche 1 bis 6,
**dadurch gekennzeichnet, dass** das Trägermaterial aus langfaserigen, linear kolliden, hocholekularen Polymeren besteht.

8. Mittel nach Anspruch 7,
**dadurch gekennzeichnet, dass** das Trägermaterial aus Polymeren mit Faserstruktur, vorzugsweise aus Proteinen besteht.

9. Mittel nach Anspruch 8,
**dadurch gekennzeichnet, dass** das Trägermaterial aus Skleroproteinen, vorzugsweise Collagenen besteht.

10. Verwendung des Mittels nach einem der Ansprüche 1 bis 9 zur Herstellung von oral oder enteral verabreichbaren Arzneimitteln, Nahrungsergänzungsmittlen oder Nahrungsmitteln mit zeitverzögerter und schonender Wirkstofffreisetzung.

## Claims

1. Composition for delayed and gentle release of active substances in the body, containing a carrier material which is insoluble or of low solubility in water and gastrointestinal fluids, active substances (4) and, where appropriate, other ancillary substances and, where appropriate, a coating which is soluble in water and gastrointestinal fluids/body fluids, **characterized in that** the foam structure of the carrier material is designed in such a way that the material
1. can be compressed without the cell walls breaking and
2. remains swellable without the cell walls being destroyed.

2. Composition according to Claim 1, **characterized in that** the active substance (4) is added to the carrier material before, during or after the production of the sponge-like structure (1, 2).

3. Composition according to either of Claims 1 or 2, **characterized in that** viscose sponges are employed as carrier material and their compressed form (1) is able to swell to a multiple (2) of its volume (1) in water or gastrointestinal fluids/body fluids.

4. Composition according to any of Claims 1 to 3, **characterized in that** the volume of the compressed carrier material (1) is not more than 2 cm³.

5. Composition according to any of Claims 1 to 4, **characterized in that** viscose sponges are employed as carrier material and swell to two to ten times, preferably four to eight times (2), their volume in the compressed state (1) in water and gastrointestinal fluids.

6. Composition according to any of Claims 1 to 5, **characterized in that** the carrier material consists of natural, semisynthetic or synthetic polymers.

7. Composition according to any of Claims 1 to 6, **characterized in that** the carrier material consists of long-fibre, linearly collidal, high molecular weight polymers.

8. Composition according to Claim 7, **characterized in that** the carrier material consists of polymers with a fibrous structure, preferably of proteins.

9. Composition according to Claim 8, **characterized in that** the carrier material consists of scleroproteins, preferably collagens.

10. Use of the composition according to any of Claims 1 to 9 for the production of medicinal compositions, food supplement compositions or food compositions, which can be administered orally or enterally, with delayed and gentle release of active substance.

## Revendications

1. Agent destiné à libérer des principes actifs dans le corps avec retard et ménagement, contenant un excipient insoluble ou difficilement soluble dans de l'eau ou dans des liquides gastro-intestinaux, des principes actifs (4) ainsi que le cas échéant d'autres adjuvants et le cas échéant un enrobage soluble dans de l'eau ou dans des liquides gastro-intestinaux/liquides corporels,
**caractérisé en ce que** la structure cellulaire de l'excipient est conformée de sorte que le matériau
1. soit compressible sans que les parois cellulaires ne cassent et
2. reste gonflable sans que les parois cellulaires ne soient détruites.

2. Agent selon la revendication 1, **caractérisé en ce que** le principe actif (4) est introduit dans l'excipient avant, pendant ou après la préparation du produit spongieux (1, 2).

3. Agent selon l'une quelconque des revendications 1 ou 2, **caractérisé en ce que** des éponges en viscose sont utilisées en tant qu'excipient, dont la forme comprimée (1) peut gonfler dans de l'eau et dans des liquides gastro-intestinaux/liquides corporels à un multiple (2) de leur volume (1).

4. Agent selon l'une quelconque des revendications 1 à 3, **caractérisé en ce que** le volume de l'excipient (1) comprimé est au maximum de 2 cm³.

5. Agent selon l'une quelconque des revendications 1 à 4, **caractérisé en ce que** des éponges en viscose sont utilisées en tant qu'excipient, qui gonflent dans de l'eau et dans des liquides gastro-intestinaux au double jusqu'au décuple, de préférence au quadruple jusqu'à l'octuple (2) de leur volume à l'état comprimé (1).

6. Agent selon l'une quelconque des revendications 1 à 5, **caractérisé en ce que** l'excipient est constitué de polymères naturels, semi-synthétiques ou synthétiques.

7. Agent selon l'une quelconque des revendications 1 à 6, **caractérisé en ce que** l'excipient est constitué de polymères à haut poids moléculaire, linéaires, colloïdaux et à longues fibres.

8. Agent selon la revendication 7, **caractérisé en ce que** l'excipient est constitué de polymères avec une structure fibreuse, de préférence de protéines.

9. Agent selon la revendication 8, **caractérisé en ce que** l'excipient est constitué de scléroprotéines, de préférence de collagènes.

10. Utilisation de l'agent selon l'une quelconque des revendications 1 à 9 pour préparer des médicaments administrables par voie orale ou entérale, des compléments alimentaires ou des produits alimentaires avec une libération de principe actif retardée et ménageante.
